# EUROPEAN PATENT SPECIFICATION

(11) **EP 0 638 321 B1**
(45) Date of publication and mention of the grant of the patent: **29.12.1999**
(21) Application number: 94111951.3
(22) Date of filing: 01.08.1994
(51) Int. Cl.: A61L 27/00, A61K 31/35, A61K 33/42, A61K 6/033, A61K 6/00

(54) **Ipriflavone and pharmaceutical compositions and their use for bone defect substitution**
Ipriflavone enthaltende pharmazeutische Zubereitungen und deren Verwendung zur Ersetzung von Knochendefekten
Compositions pharmaceutiques contenant de l'ipriflavone et leur utilisation pour la réparation des défauts osseux

(30) Priority: 02.08.1993 HU 9302230
(43) Date of publication of application: 15.02.1995
(73) Proprietor: Prezmecky, Lászlo, Dr., 4414 Füllinsdorf (CH)
(72) Inventor: Prezmeczky, Làszlo, Dr., 4414 Füllinsdorf (CH); Klenk, Dr. Gusztáv, Budapest XII (HU); Englovszky, Ms. Erzsébet, Vác (HU); Horváth, Dr. Tibor, Budapest XV (HU); Miszkiewicz, Dr.Lászlo, Budapest XIV (HU); Székács, Dr. Gábor, Budapest XIII (HU); Lévai, Dr. Ferenc, Budapest XIII (HU); Vitányi née Morvai, Mrs. Magdolna, Budapest 1054 (HU)
(74) Representative: AMMANN INGENIEURS-CONSEILS EN PROPRIETE INTELLECTUELLE SA BERNE

(56) References cited:
- EP-A- 0 129 667
- EP-A- 0 214 647
- WO-A-88/05650
- WO-A-91/14429
- WO-A-92/13538
- GB-A- 1 360 462
- BONE AND MINERAL, vol.19SUPPL., 1992, FLORENCE (ITALY) pages S27 - S33 G. MAZZUOLI ET AL. 'EFFECTS OF IPRIFLAVONE ON BONE REMODELING IN PRIMARY HYPERPARATHYROIDISM.'
- CALCIFIED TISSUE INT., vol.51, no.SUP1, 1992 pages S16 - S20 K. NOTOYA ET AL. 'STIMULATORY EFFECT OF IPRIFLAVONE ON FORMATION OF BONE-LIKE TISSUE IN RAT BONE MARROW STROMAL CELL CULTURE.'
- CALCIFIED TISSUE INT., vol.51, no.SUP1, 1992 pages S21 - S26 H. OZAWA ET AL. 'HISTOCHEMICAL AND FINE STRUCTURAL STUDY OF BONE OF IPRIFLAVONE-TREATED RATS.'

## Description

The invention relates to combinations of 7-isopropoxyisoflavone (generic name Ipriflavone) with hydroxylapatite and/or tricalcium phosphate for the substitution of pathologically developed or artificially established bone defects as well as to compositions containing these combinations, methods for their preparation, and their use.

WO-A-88/05 650 discloses dental compositions which comprise ipriflavone as anti-osteoporotic agent and an antiphlogistic agent, such as triamcinolone. In these compositions, ipriflavone exhibits an osteoclast-inhibiting activity and antagonizes the osteoclast activity increasing effects of steroidal antiphlogistics. The composltions are provided for the treatment of caries and for the healing of acute or chronic inflammatory lesions of pulp, dental radix or the bone substance surrounding them, and are formulated as dental fillers or cements, pastes for sterilizing the pulp canal, root fillers, coating compositions for protecting dental stumps, etc. .

The problem of bone defect substitution is not mentioned in this document.

During the last decade, a demand has become more and more pronounced to prevent the loss of teeth or groups of teeth instead of substituting them by removable prostheses.

Because of diseases of the periodontium, the alveolar walls and thereby the mandibles atrophize after removal or loss of a tooth or teeth being close to each other. The solid fixation of a metal implant in atrophized, bone-deficient jaws is problematic.

In the cases of certain diseases, e.g. bone tumours, the bone defect is artificially established by removing the tumour tissue, or due to a bone fracture.

It is known that porous hydroxylapatite (e.g. CEROS® 80) or tricalcium phosphate (e.g. CEROS® 82), respectively, are useful bone-substitutive materials in dentistry and maxillary surgery. The drawback of this method consists in that after filling the bone-defective region with a porous hydroxylapatite or tricalcium phosphate by suitable techniques, an amount of new bone providing a safe implantation bed has formed only after about 20 months.

Thus, it is the object of this invention to provide combinations and compositions useful to substitute bone defects of pathological or artificial genesis, (e.g. due to oral (dental) surgery, removal of bone tumours, bone fractures or the like), to accelerate the regenerative process of bone defect-substitution carried out with hydroxylapatite or tricalcium phosphate known in the art and/or to achieve new bone material adapted to the demand of locally suitable bone hardness, and to provide methods for the preparation of these combinations and compositions, and their use.

The above object is achieved according to the independent claims. The dependent claims relate to preferred embodiments.

The combinations of the present invention for substituting pathologically developed or artifically established bone defects comprise
(A) porous hydroxylapatite and/or tricalcium phosphate and
(B) 7-isopropoxyisoflavone (Ipriflavone).

These combinations comprise preferably
(A) 0.001 to 99.999 % by mass of porous hydroxylapatite and/or tricalcium phosphate, more preferably 0.01 to 99.9 % by mass, and
(B) 0.001 to 99.999 % by mass of 7-isopropoxyisoflavone (Ipriflavone), more preferably 0.1 to 99.9 % by mass,
the sum of the percentages of A and B being 100 % by mass.

These combinations may be such that they comprise two independent, separate components (A and B) which may be in the form of kits-of-parts, comprising the two separate components A and B to be mixed or to be applied separately for use. These components may be the pure active ingredients, or may comprise common excipients, carriers, vehicles, additives, etc.

The compositions of the present invention for substituting pathologically developed or artificially established bone defects comprise or consist of the above-defined combinations.

These compositions preferably comprise
(A) 0.001 to 99.999 % by mass of porous hydroxylapatite and/or tricalcium phosphate, more preferably 0.01 to 99.9 % by mass, and
(B) 0.001 to 99.999 % by mass of 7-isopropoxyisoflavone (Ipriflavone), more preferably 0.1 to 99.9 % by mass,
the sum of the percentages of A and B being 100 % by mass.

The combinations or compositions preferably comprise 10 to 90 % by mass of A and 10 to 90 % by mass of B, corresponding to a preferred mass ratio A/B of 1:9 to 9:1. More preferably, the mass ratio A/B is about 1:1.

In accordance with a preferred embodiment, the Ipriflavone is present in the form of discrete granules.

The Ipriflavone granules preferably have a particle size of about 0.1 to 1.0 mm and more preferably of about 0.5 to 0.8 mm.

In accordance with other preferred embodiments, the porous hydroxylapatite and/or the tricalciumphosphate (component A) is present in the form of discrete granules which preferably have a particle size of about 0.1 to 1.0 mm and more preferably of about 0.5 to 0.8 mm.

The Ipriflavone (B) and/or the porous hydroxylapatite and/or tricalcium phosphate (A) may be present in the form of a galenic formulation comprising common excipients, vehicles, carriers and/or additives.

The components A and/or B may be present in the form of independent, discrete formulations comprising common excipients, vehicles, carriers and/or additives.

It is known that orally aministered 7-isopropoxyisoflavone can be used for the treatment of osteoporosis.

In accordance with the above, the present invention relates to a novel use of Ipriflavone and to novel combinations thereof with porous hydroxylapatite and/or tricalcium phosphate for the substitution of bone defects of any genesis. Optionally, both components, A and B, may be present in the form of discrete granules.

Optionally, the combinations according to the invention contain the active ingredients A and B as discrete, physically separate granules, where the suitable mass ratio is adjusted by varying the amounts of the different kinds of granules. If the composition contains the active ingredients A and B according to the invention together, the composition can preferably be prepared by applying the required amount of 7-isopropoxyisoflavone onto the surface of the porous hydroxylapatite or tricalcium phosphate in a manner known per se (e.g. by wet granulation).

When 7-isopropoxyisoflavone is used in the form of discrete granules, these granules may contain, if desired, also auxiliaries (additives). Useful additives are e.g. polyvinylpyrrolidone, Avicel, Aerosil, methylcellulose, (hydroxypropyl)cellulose, (hydroxymethyl)propylcellulose, starch, mannitol or the like. The same applies to the compositions of the present invention. For the substitution of bone defects, the bone-deficient region is filled by applying suitable surgical techniques with either Ipriflavone or a composition as defined above containing porous hydroxylapatite and/or tricalcium phosphate as well as 7-isopropoxyisoflavone, optionally in the form of discrete granules, wetted by blood, serum or plasma-substitute.

The locally suitable bone hardness can be achieved by varying the mass ratio or the particle size of hydroxylapatite and/or tricalcium phosphate used in relation to 7-isopropoxyisoflavone.

In dentistry or in surgery of the jaws, respectively, it is preferred to use a combination, wherein the mass ratio of hydroxylapatite and/or tricalcium phosphate (A) to 7-isopropoxyisoflavone (B) is 1:1.

The particle size of the hydroxylapatite and/or the tricalcium phosphate is about 0.1 to 1.0 mm and preferably about 0.5 to 0.8 mm.

In accordance with another preferred embodiment, the Ipriflavone is present on the surface of the particles of component A.

The method for preparing the combinations and compositions according to the present invention is characterized by
- mixing discrete particles or granules of components A and B in the desired mixing ratio,
   or
- applying Ipriflavone (component B) onto the surface of particles or granules of component A,
optionally with use of common excipients, vehicles, carriers and/or additives.

The combination according to the invention can be used in particular for the substitution of the following bone defects. In stomatology:
- to fill the dental alveolus remaining after removal of a tooth or teeth, thereby preventing the atrophy of the walls of the dental alveolus;
- to rebuild toothless, severely atrophic dental alveoli for rehabilitation of the set of teeth by using prosthesis or implantation;
in other bone-deficient diseases:
- to substitute bone defects occurring as a consequence of pathologic, inflammatory, tumour diseases or fractures and the like.

During the use of the combination according to the invention it is advantageous to administer 7-isopropoxyisoflavone p.o. as well.

The combinations according to the invention and the results achieved by the use thereof are illustrated by the following non-limiting example.

### Example

Investigation of the direct mechanism of action of hydroxylapatite (HA) and Ipriflavone (IP) on the bone.

This experiment was carried out on female Beagle dogs of a body mass of 9 to 15 kg each in the following arrangement of groups.

| Group of dogs | Ratio of HA : IP |
|---|---|
| I | 1 : 1 |
| II | 6 : 4 |
| III | 9 : 1 |
| IV | 8 : 2 |
| V | IP alone |
| VI | HA alone |
| VII | empty bone cavities |

All groups consisted of 4 dogs each. Thus, a total of 28 dogs was used.

### Description of the surgical intervention

After a first incision made on the subjugular mucous membrane of the teeth of appropriate size, bone cavities were established entering the vestibular bone surface on the right side of the lower jaw-bone of the dogs at the site of the roots of the 4th bicuspid by amputating both roots of this tooth and closing the root canal of the amputation root stump by a retrograde root filling well established in the human dentistry.

Dimensions of the bone cavities in all dogs were:
- about 20 mm in length;
- about 8 mm in width;
- about 5 mm in depth.

The inlet orifice of the bone cavity was shaped to an elliptic form. Hydroxylapatite and Ipriflavone mixed together in a ratio corresponding to the respective group were introduced in the form of discrete granules in such a way that the amount of material soaked with blood reached the bone surface level. The wound of the mucous membrane was then tightly closed by a suture of 11 knots in average.

On the 25th day, bone tissue was taken out from all of the 28 dogs for histological examination, in some cases together with the above-lying soft tissues by using the commonly employed method of biopsy sample taking. The following results were obtained:
1. Up to a ratio of 1:1, the higher the amount of Ipriflavone was in the ratio of the two substances, the more significant were both the quality and the quantity of the newly developed bone. The bone was more compact, and its colour became more and more similar to that of the nearly intact native bone (it became more and more pale yellow), and the soft, vascularized interstitial elements (showing a reddish-brown colour) in the bone structure gradually decreased. From the point of view of ossification, the material obtained at the 1:1 mass ratio gave the best results.
2. The regions being in various phases of maturation and the borderlines thereof were very well discernible on the biopsy bone cylinder.
3. No hydroxylapatite or Ipriflavone granules were observed on the bone surfaces recovered or in the depth as well as on the surface of the biopsy cylindre.
4. In the bone cavities containing Ipriflavone alone, the newly formed bone was relatively hard and uniform but not to the same degree as the one obtained with combinations of hydroxylapatite and Ipriflavone. There were more loose tissue regions among the separate ossifying islets.
5. The consistency of tissues formed in the bone cavity and the connection thereof to the surrounding bone can be characterized as a function of the ratio used as follows.

| Consistency of the tissue | Connection to the surrounding bone | HA : JP ratio |
|---|---|---|
| Compact new bone | Rounded angles; no sharp borderline between the old and new bone | 1 : 1 |
| Loose new bone | Sharp angles with a well-pronounced borderline | 9 : 1 |
| Loose new bone with many soft, vascularized tissues | Sharp angles | only HA |
| Invaginations of soft parts | Blunted bone borders | - |

It has unambiguously been proven by the above data that the combinations according to the invention lead to an accceleration of the bone formation in comparison to the controls.

## Claims

1. Combinations for substituting pathologically developed or artificially established bone defects, comprising
(A) porous hydroxylapatite and/or tricalcium phosphate and
(B) 7-isopropoxyisoflavone (Ipriflavone).

2. Combinations according to claim 1, comprising
(A) 0.001 to 99.999 % by mass of porous hydroxylapatite and/or tricalcium phosphate, preferably 0.01 to 099.9 % by mass and
(B) 0.001 to 99.999 % by mass of 7-isopropoxyisoflavone (Ipriflavone), preferably 0.01 to 99.9 % by mass,
the sum of the percentages of A and B being 100 % by mass.

3. Combinations according to claim 1 or 2, characterized in that they are kits-of-parts, comprising the separate components A and B to be mixed or to be applied separately for use.

4. Compositions for substituting pathologically developed or artificially established bone defects, comprising or consisting of the combination according to claim 1 or 2.

5. Combinations or compositions according to one of claims 1 to 4, characterized by 10 to 90 % by mass of A and 10 to 90 % by mass of B (mass ratio A/B of 1:9 to 9:1).

6. Combinations or compositions according to one of claims 1 to 5, characterized by a mass ratio A/B of about 1:1.

7. Combinations according to one of claims 1 to 3 and 5 and 6, characterized in that the Ipriflavone is present in the form of discrete granules.

8. Combinations according to claim 7, characterized in that the Ipriflavone granules have a particles size of about 0.1 to 1.0 mm and preferably of about 0.5 to 0.8 mm.

9. Combinations according to one of claims 1 to 3 and 5 to 8, characterized in that the porous hydroxylapatite and/or the tricalcium phosphate (component A) is present in the form of discrete granules.

10. Combinations according to claim 9, characterized in that the granules of A have a particle size of about 0.1 to 1.0 mm and preferably of about 0.5 to 0.8 mm.

11. Combinations or compositions according to one of claims 1 to 10, characterized in that the Ipriflavone (B) and/or the porous hydroxylapatite and/or tricalcium phosphate (A) are present as a formulation comprising common excipients, vehicles, carriers and/or additives.

12. Combinations according to one of claims 1 to 3 and 5 to 11, characterized in that the components A and/or B are present in the form of independent, discrete formulations comprising common excipients, vehicles, carriers and/or additives.

13. Compositions according to one of claims 4 to 6 and 11, characterized in that the Ipriflavone is present on the surface of the particles of component A.

14. Method for preparing the combinations and compositions according to claims 1 to 13, characterized by
- mixing discrete particles or granules of components A and B in the desired mixing ratio, or
- applying Ipriflavone (component B) onto the surface of particles of granules of component A,
optionally with use of common excipients, vehicles, carriers and/or additives.

15. Use of the combinations or compositions according to one of claims 1 to 13 for the manufacture of medicaments for filling pathologically developed or artificially established bone deficient regions.

16. Use according to claim 15, whereby the combination or composition is introduced into the bone deficient region in the form of particles or granules wetted with blood, serum and/or serum/plasma substitute.

## Patentansprüche

1. Kombinationen zum Ersatz von pathologisch entwickelten oder künstlich erzeugten Knochendefekten, enthaltend
(A) porösen Hydroxylapatit und/oder Tricalciumphosphat, und
(B) 7-Isopropoxyisoflavon (Ipriflavon).

2. Kombinationen gemäss Anspruch 1, bestehend aus
(A) 0,001 bis 99,999 Masse-% porösem Hydroxylapatit und/oder Tricalciumphosphat, vorzugsweise 0,01 bis 099,9 Masse-%, und
(B) 0,001 bis 99,999 Masse-% 7-Isopropoxyisoflavon (Ipriflavon), vorzugsweise 0,01 bis 99,9 Masse-%,
wobei die Summe der Prozentanteile von (A) und (B) 100 Masse-% beträgt.

3. Kombinationen gemäss Anspruch 1 oder 2, dadurch gekennzeichnet, dass sie als Bestandteilsatz vorliegen, welcher aus den getrennten Komponenten (A) und (B) besteht, die zur Verwendung vermischt oder getrennt angewandt werden.

4. Zusammensetzungen zum Ersatz von pathologisch entwickelten oder künstlich erzeugten Knochendefekten, enthaltend die Kombination gemäss Anspruch 1 oder 2, oder aus ihr bestehend.

5. Kombinationen oder Zusammensetzungen gemäss einem der Ansprüche 1 bis 4, gekennzeichnet durch 10 bis 90 Masse-% von (A) und 10 bis 90 Masse-% von (B) (Massenverhältnis A/B wie 1:9 bis 9:1).

6. Kombinationen oder Zusammensetzungen gemäss einem der Ansprüche 1 bis 5, gekennzeichnet durch ein Massenverhältnis A/B von etwa 1:1.

7. Kombinationen gemäss einem der Ansprüche 1 bis 3 und 5 und 6, dadurch gekennzeichnet, dass das Ipriflavon in Form von Einzelkörnchen vorliegt.

8. Kombinationen gemäss Anspruch 7, dadurch gekennzeichnet, dass die Ipriflavonkörnchen eine Teilchengrösse von etwa 0,1 bis 1,0 mm und vorzugsweise von etwa 0,5 bis 0,8 mm aufweisen.

9. Kombinationen gemäss einem der Ansprüche 1 bis 3 und 5 bis 8, dadurch gekennzeichnet, dass der poröse Hydroxylapatit und/oder das Tricalciumphosphat (Komponente A) in Form von Einzelkörnchen vorliegt.

10. Kombinationen gemäss Anspruch 9, dadurch gekennzeichnet, dass die Körnchen von A eine Teilchengrösse von etwa 0,1 bis 1,0 mm und vorzugsweise von etwa 0,5 bis 0,8 mm aufweisen.

11. Kombinationen oder Zusammensetzungen gemäss einem der Ansprüche 1 bis 10, dadurch gekennzeichnet, dass das Ipriflavon (B) und/oder der poröse Hydroxylapatit und/oder das Tricalciumphosphat (A) als Formulierung vorliegen, welche übliche Excipientien, Trägerstoffe und/oder Additive enthält.

12. Kombinationen gemäss einem der Ansprüche 1 bis 3 und 5 bis 11, dadurch gekennzeichnet, dass die Komponenten A und/oder B als unabhängige, getrennte Formulierungen vorliegen, welche übliche Excipientien, Trägerstoffe und/oder Additive enthalten.

13. Zusammensetzungen gemäss einem der Ansprüche 4 bis 6 und 11, dadurch gekennzeichnet, dass das Ipriflavon an der Oberfläche der Teilchen der Komponente A vorliegt.

14. Verfahren zur Herstellung der Kombinationen und Zusammensetzungen gemäss Ansprüchen 1 bis 13, gekennzeichnet durch
- Vermischen einzelner Teilchen oder Körnchen der Komponenten A und B im gewünschten Mischungsverhältnis, oder
- Aufbringen von Ipriflavon (Komponente B) auf die Oberfläche von Teilchen oder Körnchen der Komponente A,
gegebenenfalls unter Verwendung üblicher Excipientien, Trägerstoffe und/oder Additive.

15. Verwendung der Kombinationen oder Zusammensetzungen gemäss einem der Ansprüche 1 bis 13 zur Herstellung von Medikamenten zur Füllung pathologisch entwickelter oder künstlich erzeugter Knochendefektstellen.

16. Verwendung gemäss Anspruch 15, wobei die Kombination oder Zusammensetzung in die Knochendefektstellen als Teilchen oder Körnchen eingebracht wird, welche mit Blut, Serum und/oder Serum/Plasmaersatz benetzt sind.

## Revendications

1. Combinaisons pour substituer des défauts d'os pathologiquement développés ou artificiellement établis, comprenant
(A) de l'hydroxylapatite poreuse et/ou du phosphate de tricalcium, et
(B) de la 7-isopropoxyisoflavone (ipriflavone).

2. Combinaisons selon la revendication 1, comprenant
(A) de 0,001 à 99,999 % en masse d'hydroxylapatite poreuse et/ou de phosphate de tricalcium, de préférence de 0,01 à 099,9 % en masse, et
(B) de 0,001 à 99,999 % en masse de 7-isopropoxyisoflavone (ipriflavone), de préférence de 0,01 à 99,9 % en masse,
la somme des pourcentages de (A) et de (B) étant de 100 % en masse.

3. Combinaisons selon la revendication 1 ou 2, caractérisées en ce qu'elles sont des kits de composants, comprenant les composants (A) et (B) séparés pour être mélangés ou pour être appliqués séparément lors de l'utilisation.

4. Combinaisons pour substituer des défauts d'os développés pathologiquement ou établis artificiellement, comprenant ou consistent en la combinaison selon la revendication 1 ou 2.

5. Combinaisons ou compositions selon l'une quelconque des revendications 1 à 4, caractérisées par 10 à 90 % en masse de (A) et 10 à 90 % en masse de (B) (rapport de masse A/B de 1:9 à 9:1).

6. Combinaisons ou compositions selon l'une quelconque des revendications 1 à 5, caractérisées par un rapport de masse A/B d'environ 1:1.

7. Combinaisons selon l'une quelconque des revendications 1 à 3 et 5 et 6, caractérisées en ce que l'ipriflavone est présente sous forme de granulés discrets.

8. Combinaisons selon la revendication 7, caractérisées en ce que les granulés d'ipriflavone présentent une grosseur de particules d'environ 0,1 à 1,0 mm et de préférence d'environ 0,5 à 0,8 mm.

9. Combinaisons selon l'une quelconque des revendications 1 à 3 et 5 à 8, caractérisées en ce que l'hydroxylapatite poreuse et/ou le phosphate de tricalcium (composant A) sont présents sous forme de granulés discrets.

10. Combinaisons selon la revendication 9, caractérisées en ce que les granulés de A présentent une grosseur de particules d'environ 0,1 à 1,0 mm et de préférence d'environ 0,5 à 0,8 mm.

11. Combinaisons ou compositions selon l'une quelconque des revendications 1 à 10, caractérisées en ce que l'ipriflavone (B) et/ou l'hydroxylapatite poreuse et/ou le phosphate de tricalcium (A) sont présents sous forme de formulation comprenant des excipients usuels, des véhicules, des supports et/ou des additifs.

12. Combinaisons selon l'une quelconque des revendications 1 à 3 et 5 à 11, caractérisées en ce que les composants A et/ou B sont présents sous forme de formulation indépendante et discrète comprenant des excipients usuels, des véhicules, des supports et/ou des additifs.

13. Compositions selon l'une quelconque de revendications 4 à 6 et 11, caractérisées en ce que l'ipriflavone est présente sur la surface des particules du composant A.

14. Procédé pour la préparation des combinaisons et compositions selon les revendications 1 à 13, caractérisé par
- le mélange de particules ou granulés discrets des composants A et B dans le rapport de mélange désiré, ou
- l'application de l'ipriflavone (composant B) sur la surface de particules ou granulés du composant A,
éventuellement avec l'utilisation d'excipients, de véhicules, de supports et/ou d'additifs usuels.

15. Utilisation des combinaisons ou compositions selon l'une quelconque des revendications 1 à 13 dans la préparation de médicaments pour obturer des régions défectueuses d'os développées pathologiquement ou établies artificiellement.

16. Utilisation selon la revendication 15, dans laquelle la combinaison ou la composition est introduite dans la région défectueuse de l'os sous forme de particules ou granulés, mouillés avec du sang, du sérum et/ou d'un succédané de sérum/plasma.
